# EUROPEAN PATENT APPLICATION

(11) **EP 2 583 986 A1**
(43) Date of publication of application: **24.04.2013**
(21) Application number: 11185460.0
(22) Date of filing: 17.10.2011
(51) Int. Cl.: C08F 290/06, C08G 18/48, C08G 18/67, C09D 175/16

(54) **Fluorinated water-oil repellency agents**

(71) Applicant: Cytec Surface Specialties, S.A., 1070 Brussels (BE)
(72) Inventor: Cleymans, Ruben, 1500 Halle (BE); Cappelle, Steven, 9400 Ninove (BE); Hutchins, Marcus Lee, Hiram GA Georgia 30141 (US); Moens, Luc, 1640 Sint-Genesius-Rode (BE)
(74) Representative: Schoofs, Hilde

(57) **Abstract**

The present invention relates to an active energy ray curable compound **(A)** comprising: at least one active energy ray curable group, at least one fluorine-containing moiety **(a1)** and at least one moiety **(a2)** comprising at least one portion represented by Formula (1):

-[C(=O)-CH(R)-O-C(=O)-CH(R)-O]n-

wherein n is an integer from 1 to 10, and wherein R is selected from -H or -CH₃,

The invention further relates to ways of making such compounds and to their use in providing water-oil repellency properties to a coating composition.

## Description

The present invention relates to fluorinated compounds showing very good miscibility and compatibility with an active energy ray curable resin matrix, to their production and their use in applications that require amongst others excellent stain resistance and fingerprint removability. Smears such as fingerprints but also sebum, sweat and cosmetics tend to easily stick to e.g. hard coat films or stain resistant layers; and once they happen to stick, the smears tend to be difficult to remove. In particular on optical members with an antireflection film, the attached stains or smears stand out and are thereby problematic in various ways. First there is the aesthetic effect. When attempting to wipe off any smears with an ordinary paper or woven cloth, they rather spread out to soil the entire surface. Further there is the risk of damaging the hard coat or the stain resistant layer by doing so.

Generally, when a hard coat surface is provided with an easy-to-clean property a low surface energy additive is added (e.g. a silicone or fluoro additive).

Unfortunately standard fluorinated compounds tend to have a low affinity for commercial resins of a standard coating formulation leading to phase separation, low transparency and/or poor film formation. To increase the miscibility/compatibility between the fluorinated compound and the coating resin matrix, a compatibilizing group can be linked to the fluorinated compound. US7632874 describes the use of fluorine-containing perfluoroether urethane acrylates in a radiation curable formulation. Since these compounds do not comprise a compatibilizing group there is limited compatibility with the non-fluorine containing resin matrix.

Against this background we now provide an active energy ray curable compound (A) comprising at least one fluorine-containing moiety **(a1)** and at least one moiety **(a2)** comprising at least one portion represented by Formula (1):

-[C(=O)-CH(R)-O-C(=O)-CH(R)-O]n-

wherein n is an integer from 1 to 10, and wherein each of R is selected from -H or -CH₃. Typically n is an integer from 1 to 5, more typically from 2 to 4.

More in particular there is provided an active energy ray curable compound **(A)** comprising at least one fluorine-containing moiety **(a1)** and at least one (poly)lactide- and/or (poly)glycolide-containing moiety **(a2).**

Compounds **(A)** of the invention typically comprise at least one, and more typically at least two active energy ray curable groups per molecule. By "active energy ray curable groups" is meant functional groups which under the influence of irradiation with active energy rays and/or a (photo)initiator can undergo polymerization. Examples of such groups include (meth)acryloyl groups, vinyl group, allyl groups, thiol groups and/or epoxy groups.

Most typically the active energy ray curable groups are polymerizable ethylenically unsaturated groups. By "polymerizable ethylenically unsaturated groups" is meant to designate in the present invention carbon-carbon double bonds which under the influence of irradiation and/or a (photo)initiator can undergo radical polymerization. The polymerizable ethylenically unsaturated groups are generally chosen from (meth)acrylyol groups and/or allyl groups, preferably they are (meth)acryloyl groups, most preferably acryloyl groups. In the present invention, the term "(meth)acryloyl' is to be understood as to encompass both acryloyl and methacryloyl groups or derivatives as well as mixtures thereof.

In general moieties **(a1)** are fluorine-containing moieties. Moieties **(a1)** advantageously are capable of exerting water and/or oil repellency. Typically moieties **(a1)** comprise at least one fluorine-containing organic group, more in particular at least one fluorine-containing organic group of a fluorine-containing compound which is generally used as a water repellent and/or oil repellent. Preferably moieties **(a1)** are selected from one or more of the following: a moiety containing one or more polyfluoroalkyl groups, a fluorinated alcohol-derived moiety and/or a fluorosilicone-derived moiety.

Examples of suitable compounds that may be used in the present invention include but are not limited to homopolymers of a polymerizable monomer containing a polyfluoroalkyl group such as a (meth)acrylate containing a polyfluoroalkyl group or a copolymer of such a monomer with another polymerizable monomer such as an acrylate, maleic anhydride, chloroprene, butadiene or methyl vinyl ketone; polyfluorinated oxetane alcohols such as those described in WO2001000701; hydroxyl functional fluoroethylene-alkyl vinyl ethers such as those described in (US 20110028612); fluorinated (poly)ether alcohols and/or hydroxyl functional fluorosilicone compounds. Such compounds are well known in the art.

Preferred in the frame of the present invention are moieties **(a1)** that are derived from fluorinated alcohols. Examples of suitable fluorinated alcohols are fluorinated oxetane alcohols, hydroxyl functional fluoroethylene-alkyl vinyl ethers, fluoroalkyl alcohols and/or fluorinated (poly)ether alcohols. For more examples of suitable fluorinated alcohols - see compounds (i) and (i') below.

Particularly preferred are fluorinated (poly)ether alcohol moieties, in particular polyfluoro(poly)ether alcohol moieties, more in particular perfluoro(poly)ether alcohol moieties **(a1).** The "term (poly)ether" covers both ethers and polyethers comprising a plurality of ether groups, as well as mixtures of both.

Most typically moieties **(a1)** comprise at least one portion represented by one or more of the Formulae (2) to (6):

-(CF₂CF₂O)p- Formula (2)

-(CF₂CF(CF₃)O)q-; Formula (3)

-(CF₂CF₂CF₂0)r- Formula (4)

-(CF₂O)s- Formula (5)

-(CkF2k+1)- Formula (6);

wherein k is an integer of from 1 to 16, and
wherein each of p, q, r and s is an integer of from 1 to 100, preferably from 1 to 80. When each of p, q, r and s is within the above range, the surface of the coating film after curing will be excellent in water and/or oil repellency, and excellent fingerprint removability will be maintained over a long period. Even more preferred are moieties **(a1)** that comprise at least one portion represented by one or more of the Formulae (2) to (5) as identified above.

Typically the weight percentage (wt%) of moieties **(a1),** relative to the total weight of the compound **(A),** is from 1 to 90 wt%. In general their amount is at least 30 wt%, preferably at least 40 wt%. Often their amount is at most 80 wt%, typically at most 70 wt%.

Typically moieties **(a2)** are (poly)lactide- and/or (poly)glycolide-containing moieties. By (poly)lactide is meant a lactide or a polylactide. By (poly)glycolide is meant a glycolide or a polyglycolide. In one variant of the invention; moieties **(a2-1)** are (poly)lactide-containing moieties. In another variant of the invention moieties **(a2-2)** are (poly)glycolide-containing moieties. In a third variant of the invention, both types of moieties **(a2)** are present in compounds **(A).** For more examples of suitable (poly)lactide- and/or (poly)glycolide-containing compounds - see compounds **(iii')** below.

Preferred in the frame of the invention are (poly)lactide-containing moieties **(a2-1).** Particularly suitable are moieties **(a2-1)** that comprise at least one portion:

-[C(=O)-CH(R)-O-C(=O)-CH(R)-O]n- Formula (7),

wherein n is an integer from 1 to 10, and wherein R is -CH₃. Optionally moieties **(a2-2)** can be present as well, more in particular moieties **(a2-2)** that comprise at least one portion:

-[C(=O)-CH(R)-O-C(=O)-CH(R)-O]n- Formula (8),

wherein n is an integer from 1 to 10, and wherein R is -H. Typically in any of these, n is an integer from 1 to 5, more typically from 2 to 4.

The (poly)lactide and/or (poly)glycolide groups, and in particular the (poly)lactide groups provide a very good miscibility and compatibility with an active energy ray curable resin matrix. Typically the weight percentage (wt%) of moieties **(a2),** relative to the total weight of the compound **(A),** is from 5 to 75 wt%. In general their amount is at least 7 wt%, preferably at least 10 wt%. Often their amount is at most 60 wt%, typically at most 50 wt%.

The active energy ray curable groups can be provided by a further moiety **(a3).** By "further" is meant that this moiety is different from moieties **(a1)** and **(a2).** Examples of suitable compounds for use in the present invention are for instance compounds **(vi')** specified below.

In a particular embodiment of the invention the active energy ray curable groups can also be provided by said same moiety **(a2).** Examples of suitable moieties **(a2')** are for instance those derived from active energy ray curable compounds that contain at least one (preferably essentially one) reactive group capable to react with e.g. isocyanate groups and at least one portion:

-[C(=O)-CH(R)-O-C(=O)-CH(R)-O]n- ,

wherein n and R are as specified above (see Formulas (1), (7) and (8) respectively). Throughout the invention and unless stated otherwise by "reactive group capable to react with isocyanate groups" in general is meant a hydroxyl group. Other possible groups are amino or thiol groups. Hydroxyl groups are preferred. For examples of suitable compounds that may provide moieties **(a2')** - see compounds (iii) below.

Typically the weight percentage (wt%) of such moieties **(a2'),** relative to the total weight of the compound **(A),** is from 15 to 80 wt%. In general their amount is at least 20 wt%, preferably at least 30 wt%. Often their amount is at most 75 wt%, typically at most 60 wt%.

Compounds **(A)** of the invention may typically comprise at least one other moiety **(a4).** By "other" is meant that this moiety is different from moieties **(a1), (a2), (a2')** or **(a3).**

The position of the (poly)lactide and/or (poly)glycolide units in compounds **(A)** of the invention is not really important. Compounds **(A)** can be prepared in various different ways. Below some preferred ways to prepare compounds **(A)** of the invention are described.

In a first embodiment of the invention, compounds **(A)** may be obtained from the reaction of: at least one fluorinated alcohol **(i),** optionally, at least one alcohol **(iv)** different from **(i),** at least one polyisocyanate **(ii),** and at least one (poly)lactide- and/or (poly)glycolide-modified compound **(iii)** containing at least one active energy ray curable group and at least one (preferably essentially one) reactive group capable to react with isocyanate groups. Preferably compound **(iii)** is a (poly)lactide-modified compound.

The optional compounds **(iv)** may replace up to 75% by weight of compounds **(i).** Often this amount is at most 50 wt%, more often at most 25 wt%. In a first variant of this embodiment, no compounds **(iv)** are used to prepare compounds **(A)** of the invention. In a second variant, compounds **(iv)** are used to prepare compounds **(A)** of the invention. In a third variant a mixture of both types of compounds **(A)** may be used in the frame of the present invention.

The optional compounds **(iv)** typically are hydrophobic hydroxyl functional compounds including but not limited to hydroxyl functional alkoxysilanes such as described in US 20060102050 and/or fatty alcohols (typically C7-C22) such as lauryl, myristyl, cetyl, stearyl described in e.g. US20110048281 and/or long chain alkyl alcohols (typically C6-C35) such as described in e.g. US 20110008634 and US 20100215775.

Typically compounds **(A)** according to this first embodiment are obtained via a process comprising:
- a first step comprising the reaction of at least one fluorinated alcohol (compound i) and at least one polyisocyanate (compound **ii**) to form an NCO terminated (poly)urethane prepolymer, and
- a second step comprising the reaction of the product of the first step with at least one (poly)lactide- and/or (poly)glycolide-modified compound **(iii)** containing at least one active energy ray curable group and at least one (preferably essentially one) reactive group capable to react with isocyanate groups. Alternatively, the first step may comprise the reaction of at least one compound **(iii)** with at least one compound **(ii)** and the second step then comprises the further reaction with at least one compound **(i).** In each of these variants compound **(iii)** typically is a (poly)lactide-modified compound. In each of these variants the at least one active energy ray curable group typically is a (meth)acryloyl group. Typically compounds **(iii)** are end-capping agents. Optionally, at least one compound **(iv)** can be used in admixture with at least one compound **(i).**

The present invention relates to the above described processes for preparing compounds **(A)** as well as to compounds **(A)** obtainable via said processes. Preferred process conditions and preferred compounds **(i)** to **(iii)** and where present **(iv)** are described below.

The process of the invention can be carried out by reacting a stoechiometric excess of compounds **(ii)** with compounds **(i)** and optionally **(iv),** preferably under substantially anhydrous conditions and at a temperature between 30°C and 130°C, more preferably between 70°C and 100°C, until the reaction between the isocyanate groups and the isocyanate-reactive groups is substantially complete. The isocyanate content can be followed by titration with an amine. The reactants are generally used in proportions corresponding to an equivalent ratio of isocyanate groups provided by compound **(ii)** to isocyanate-reactive groups provided by compounds **(i)** and where present **(iv)** of from about 1.5 to about 4, preferably from about 1.8 to about 2.5. The reaction may be facilitated by the addition of about 5 to about 40%, preferably about 15 to about 25%, by weight of a solvent in order to reduce the viscosity of the pre-polymer. The solvent is preferably acetone or methylethylketone. During this process, it is common to use catalysts to accelerate the reaction of the isocyanates towards isocyanate-reactive groups (e.g. hydroxyls) and to use inhibitors in order to prevent the radical reaction of the reactive unsaturations. It is possible in the frame of this invention to use a sequential process during which compound **(i)** and/or compounds **(ii)** and/or compounds **(iv)** are added incrementally in two or several portions, or with a continuous feed. The reason for this is a better control on the exothermicity of the reaction, especially when no solvent is present.

In a subsequent step, the isocyanate-terminated polyurethane pre-polymer is reacted with one or more compounds **(iii),** preferably in the same conditions as for the previous step. The reactants are generally used in proportions corresponding to an equivalent ratio of isocyanate groups provided by the pre-polymer obtained in the first step to isocyanate-reactive groups provided by compound **(iii)** of from about 0.5 to about 1.5, preferably from about 0.7 to about 1.2. The isocyanate content can be followed by titration with an amine.

Compounds **(i)** typically are fluorinated alcohols that may be selected from fluoroalkyl alcohols, fluorinated oxetane alcohols (more in particular perfluoro oxetane alcohols), hydroxyl functional fluoroethylene-alkyl vinyl ethers (more in particular perfluoroalkylvinylether alcohols) and/or fluorinated (poly)ether alcohols (more in particular perfluoro(poly)ether alcohols).

Fluorinated (poly)ether alcohols, in particular polyfluoro(poly)ether alcohols, and more in particular perfluoro(poly)ether compounds containing one or more hydroxyl groups are preferred. Preferred are compounds **(i)** that comprise at least one portion represented by one ore more of the Formulae (2) to (6):

-(CF₂CF₂O)p- Formula (2)

-(CF₂CF(CF₃)O)q-; Formula (3)

-(CF₂CF₂CF₂O)r- Formula (4)

-(CF₂O)s- Formula (5)

-(CkF2k+1)- Formula (6);

wherein k, p, q, r and s are as defined above. Preferred are portions according to any of Formulae (2) to (5). Most typically these compounds **(i)** have a hydroxyl functionality between 1 and 4, more typically between 1 and 2. Preferred are diols.

Examples of suitable compounds **(i)** include but are not limited to: Fluorolink® E, Fluorolink® E10, Fluorolink® E10H, Fluorolink® D, Fluorolink® D10, Fluorolink® D10H, Fluorolink® T10, and Fluorolink® T from Solvay Solexis, Demnum-SA from Daikin, Krytox® OH from Dupont^{™}, LUMIFLON® LF200F and LUMIFLON® LF916F from AGC Chemicals, PolyFox® PF-636 PolyFox® PF-6320, PolyFox® PF-656, PolyFox® PF-6520 and PolyFox® PF-7002 from OMNOVA, and mixtures thereof (of any of these).

The amount of compounds **(i)** used for the synthesis of compounds **(A)** is generally in the range of from 1 to 90 wt%. Preferably their amount is at least 30 wt%, and typically at least 40 wt%. In general their amount is at most 80 wt%, and typically at most 70 wt%, relative to the total weight of the compound **(A).**

By a "polyisocyanate" (compound **ii**) in the present invention is meant to designate an organic compound comprising at least two isocyanate groups. The polyisocyanate usually comprises not more than three isocyanate groups. The polyisocyanate compound most preferably is a diisocyanate. Polyisocyanates may be selected from one or more aliphatic, cycloaliphatic, aromatic and/or heterocyclic polyisocyanates well known in the art. Examples of aliphatic and cycloaliphatic polyisocyanates that may be used are: 1,6-diisocyanatohexane (HDI), 1,1'-methylene bis[4-isocyanatocyclohexane] (H12MDI), 5-isocyanato-1-isocyanatomethyl-1,3,3-trimethylcyclohexane (isophorone diisocyanate, IPDI). Aliphatic polyisocyanates containing more than two isocyanate groups are for example the derivatives of above mentioned diisocyanates like 1,6-diisocyanatohexane biuret and isocyanurate. Examples of aromatic polyisocyanates that may be used are 1,4-diisocyanatobenzene (BDI), 2,4-diisocyanatotoluene (TDI), 1,1'-methylenebis[4-isocyanatobenzene] (MDI), xylilene diisocyanate (XDI), 1,5-naphtalene diisocyanate (NDI), tolidine diisocyanate (TODI), tetramethylxylylene diisocyanate (TMXDI) and p-phenylene diisocyanate (PPDI). Preferred are aliphatic polyisocyanates, most preferred are aliphatic diisocyanates.

The amount of compounds **(ii)** used for the synthesis of compounds **(A)** is generally in the range of from 5 to 75 wt%. Preferably their amount is at least 7 wt%, and typically at least 10 wt%. In general their amount is at most 60 wt%, and typically at most 40 wt%, relative to the total weight of the compound **(A).**

Preferably (poly)lactide- and/or (poly)glycolide-modified compounds **(iii)** contain at least one active energy ray curable group and essentially one reactive group capable to react with isocyanate groups. By (poly)lactide-modified compounds is meant to designate compounds containing lactide units or polylactide units. By (poly)glycolide-modified compounds is meant to designate compounds containing glycolide units or polyglycolide units. Typically compounds **(iii)** comprise at least one portion corresponding to Formula (1) as defined above.

An example of suitable compounds **(iii)** are the reaction products (or adducts) of **(iii')** a lactide and/or a glycolide and **(vi')** a hydroxy (meth)acrylate comprising at least one hydroxyl group, preferably a hydroxyalkyl (meth)acrylate, wherein the reaction product contains on average one (or essentially one) hydroxyl functional group capable to react with e.g. isocyanate groups. The amount of hydroxy (meth)acrylates, in particular hydroxyalkyl (meth)acrylates **(vi')** used for the synthesis of compounds **(iii)** is generally in the range from 5 to 80 wt%. Preferably their amount is at least 10 wt% and typically at least 15 wt%. In general their amount is at most 70 wt%, and typically at most 60 wt%, relative to the total weight of the compound **(iii).** Preferred molecules in this category are hydroxymethyl (meth)acrylate, hydroxyethyl (meth)acrylate, hydroxypropyl (meth)acrylate and/or hydroxybutyl (meth)acrylate. Preferably compounds **(iii')** are actides. More details on suitable compounds **(iii')** and **(vi')** are given below.

Suitable compounds **(iii)** are in particular those represented by Formula (9):

CH2=C(R')C(=O)O(CrH2rO)t-[C(=O)-CH(R)-O-C(=O)-CH(R)-O]n-H

wherein r is an integer from 1 to 4, t is an integer from 1 to 4, n is an integer from 1 to 10, and wherein each of R and R', independently, is selected from -H or -CH₃. Preferably R is -CH3. Particularly suited compounds **(iii)** are hydroxyC₁₋₄alkoxy(meth)acrylate-((poly)lactide)ₙ compounds, wherein n is an integer between 1 and 10, preferably n is between 1 and 5 and most preferably n is between 2 and 4.

The amount of compounds **(iii)** used for the synthesis of compounds **(A)** is generally in the range of from 5 to 75 wt%. Typically their amount is at least 7 wt%, more typically at least 10 wt%. Generally their amount is at most 60 wt%, preferably at most 40 wt%, relative to the total weight of the compound **(A).**

In a second embodiment of the invention, compounds **(A)** may be obtained from the reaction of: at least one fluorinated alcohol **(i'),** optionally, at least one alcohol **(iv')** different from **(i'),** at least one polyisocyanate **(ii'),** at least one lactide and/or glycolide **(iii'),** and at least one compound **(vi')** containing at least one active energy ray curable group and at least one (preferably essentially one) reactive group capable to react with isocyanate groups.

Typically compounds **(A)** according to this second embodiment are obtained via a process comprising: a first step comprising the reaction of at least one fluorinated alcohol (compound **i'**) with at least one lactide and/or glycolide (compound **iii**') that adds to the alcohol in a ring opening reaction. Typically in this first step a prepolymer is formed that contains at least one hydroxyl group. The process of the invention further comprises a second step comprising the reaction of the product of the first step with at least one polyisocyanate (compound **ii'**) to form an NCO terminated (poly)urethane prepolymer, and a third step comprising the reaction of the product of the second step with at least one compound **(vi')** containing at least one active energy ray curable group and at least one (preferably essentially one) reactive group capable to react with isocyanate groups. Alternatively, the first step may comprise the reaction of at least one compound **(vi')** with at least one compound **(ii')** and the second step then comprises the further reaction of the product formed in step 1 with the reaction product of at least one compound **(i')** and at least one lactide and/or glycolide (compound **iii'**). In each of these variants, compound **(iii')** preferably is a lactide. In each of these variants the at least one active energy ray curable group typically is a (meth)acryloyl group. Optionally, at least one compound **(iv')** can be used in admixture with at least one compound **(i').**

The present invention relates to the above described processes for preparing compounds **(A)** as well as to compounds **(A)** obtainable via said processes.

Compounds **(i'), (ii')** and **(iv')** may be selected from the same list of compounds as given for compounds **(i), (ii)** and **(iv)** above, and unless specified otherwise they are used in similar amounts. Reaction conditions unless specified otherwise are also the same as indicated above. The lactides and/or glycolides **(iii')** used may be derived from a renewable resource such as corn, sugar beet or cheese whey.

Examples of suitable compounds **(iii')** for use in the present invention include but are not limited to Galacid Slow release from GALACTIC SA, FUTERRO® Lactide LF from Futerro, PURALACT® L, PURALACT® D or PURASORB® G from Purac, or mixtures of these (of any of these).

The amount of compounds **(iii')** used for the synthesis of compounds **(A)** of the invention is generally in the range of from 5 to 50 wt%. Typically their amount is at least 7 wt%, more typically at least 10 wt%. Generally their amount is at most 40 wt%, preferably at most 25 wt%, relative to the total weight of the compound **(A).**

Typically compounds **(vi')** are end-capping agents comprising essentially one reactive group capable to react with isocyanate groups. By compounds **(vi')** containing at least one active energy ray curable group and essentially one reactive group capable to react with isocyanate groups is meant to designate in the present invention compounds comprising at least one active energy ray curable function such as acryloyl and/or methacryoyl groups and one nucleophilic function capable of reacting with isocyanate, preferably an hydroxyl group. Preferred are (meth)acryloyl mono-hydroxy compounds, though poly(meth)acryloyl mono-hydroxy compounds may also be used. Acrylates are particularly preferred.

Useful compounds **(vi')** include the esterification products of aliphatic and/or aromatic polyols with (meth)acrylic acid having a residual average hydroxyl functionality of about 1. The partial esterification products of (meth)acrylic acid with tri-, tetra-, penta- or hexahydric polyols or mixtures thereof are preferred. In this context, it is also possible to use reaction products of such polyols with ethylene oxide and/or propylene oxide or mixtures thereof, or reaction products of such polyols with lactones, which add to these polyols in a ring-opening reaction. Examples of suitable lactones are γ-butyrolactone and, in particular δ-valerolactone and ε-caprolactone. These modified or unmodified polyols are partly esterified with acrylic acid, methacrylic acid or mixtures thereof until the desired residual hydroxyl functionality is reached.

Compounds (vi') obtained from the reaction of (meth)acrylic acid with aliphatic, cycloaliphatic or aromatic compounds bearing an epoxy functionality together with at least one (meth)acrylic functionality can be used as well. Other suitable compounds **(vi')** are the (meth)acrylic esters with linear and branched polyols in which at least one hydroxy functionality remains free, like hydroxyalkyl(meth)acrylates having 1 to 20 carbon atoms in the alkyl group. Preferred molecules in this category are hydroxymethyl (meth)acrylate, hydroxyethyl (meth)acrylate, hydroxypropyl (meth)acrylate and/or hydroxybutyl (meth)acrylate. Particularly preferred are also compounds comprising at least two (meth)acryl functions such as glycerol diacrylate, trimethylolpropane diacrylate, glycerol diacrylate, pentaerythritol triacrylate, ditrimethylolpropane triacrylate, dipentaerythritol pentaacrylate and their (poly)ethoxylated and/or (poly)propoxylated equivalents.

The amount of compounds **(vi')** used for the synthesis of compounds **(A)** of the invention is generally in the range of from 3 to 50 wt%. Typically their amount is at least 5 wt%, more typically at least 7 wt%. Generally their amount is at most 50 wt%, preferably at most 45 wt%, relative to the total weight of the compound **(A).**

In a third embodiment of the invention, compounds **(A)** may be obtained from the reaction of: at least one fluorinated alcohol **(i'),** optionally, at least one alcohol **(iv')** different from **(i'),** at least one lactide and/or glycolide **(iii'),** at least one anhydride **(vii'),** and at least one compound **(viii')** containing at least one active energy ray curable group and at least one (preferably essentially one) reactive group capable to react with carboxylic acid groups.

Typically compounds **(A)** according to this third embodiment are obtained via a process comprising: a first step that is the same as for the second embodiment. The process of the invention further comprises a second step comprising the reaction of the product of the first step with at least one anhydride (compound **vii**') to form an carboxylic acid terminated prepolymer, and a third step comprising the reaction of the product of the second step with at least one (meth)acrylated compound **(viii')** containing at least one (preferably essentially one) reactive group capable to react with carboxylic acid groups.

The present invention relates to the above described processes for preparing compounds **(A)** as well as to compounds **(A)** obtainable via said processes.

Compounds **(vii')** typically are anhydrides. Examples of suitable anhydrides include but are not limited to succinic anhydride, maleic anhydride, phthalic anhydride, trimellitic anhydride and/or pyromellitic dianhydride. Preferred are succinic anhydride, maleic anhydride and/or phthalic anhydride. The reactive groups of compounds **(viii')** capable to react with carboxylic acid groups may be selected from hydroxyl groups, epoxy groups and/or amino groups. Preferred are epoxy groups. A particularly preferred compound **(viii')** is glycidyl (meth)acrylate.

Compounds **(A)** prepared according to the first and/or second embodiment are in general preferred. Typically compounds **(A)** of the invention are (poly)lactide- and/or (poly)glycolide-modified fluoro urethane (meth)acrylates. More typically compounds **(A)** of the invention are (poly)lactide-modified fluoro urethane (meth)acrylates.

Compounds **(A)** of the invention typically are characterized by a weight average molecular weight (Mw) of from 500 to 50,000 Daltons, preferably from 500 to10,000 Daltons.

Molecular weights in the invention typically are typically determined by conventional gel permeation chromatography (GPC) using polystyrene standards (typically in the Molecular Weight range: 200 - 7.500.000 Daltons).

Compounds **(A)** of the invention typically are characterized by a viscosity as determined according to DIN EN ISO 3219 in the range of from 500 mPa.s at 25°C to 1,000,000 mPa.s at 60°C. Preferred is a viscosity of from 1,000 mPa.s at 25°C to 500,000 mPa.s at 60°C.

Besides providing hydrophobicity, compounds **(A)** of the invention are capable of one or more of the following: imparting chemical stability, wheatering resistance, release properties, high temperature resistance, barrier properties, low coefficients of friction, water impermeability, corrosion resistance, low refractive index etc. to coatings prepared from such materials. Non-wettable surfaces may further also impart the ability to prevent frost and/or ice forming or adhering to the surface.

Compounds **(A)** of the invention were found to exhibit excellent miscibility and compatibility with radiation curable resin matrices standard in the art. Compounds **(A)** of the invention further are highly suitable for use in coating compositions that require excellent stain resistance and fingerprint removability.

Provided as such is also a composition, in particular an active energy ray curable composition, more in particular a radiation curable composition comprising at least one compound **(A)** according of the invention.

Typically compositions of the invention may further comprise at least one other curable compound **(B)** containing at least one active energy ray curable group. The active energy curable groups of compounds **(B)** typically are selected from (meth)acryloyl groups, allyl groups and/or vinyl groups. Most typically they are (meth)acryloyl groups. By "other" is meant that it is different from compounds **(A).**

Typically compositions of the invention comprise, relative to the total weight of the organic non-volatile content of the composition, from 0.01 to 100 wt% of compounds **(A).** In general this amount is at least 0.05 wt%, typically at least 0.1 wt% of compounds **(A).** Usually, this amount is at least 1 wt%, preferably this amount is at least 2 wt%, more preferably at least 3 wt%, even more preferably at least 5 wt%. In general this amount is at most 99.5 wt%, often at most 99 wt%. Typically this amount is at most 98 wt%, more typically at most 97 wt%, even more typically at most 90 wt%. Often however this amount is at most 50 wt%, more often at most 30 wt%.

Typically compositions of the invention comprise, relative to the total weight of the organic non-volatile content of the composition, from 0 to 99.99 wt%, more in particular from 0.01 to 99.99 wt% of compounds **(B).** Where present, they are typically present in an amount of at least 50 wt%, more typically at least 70 wt%, relative to the total weight of the composition. Typically this amount is at most 99.95 wt%, more typically at most 99.90 wt%, in general at most 97 wt%, most preferably at most 95 wt%.

Typically, compositions of the invention comprise, relative to the total weight of the organic non-volatile content of the composition, from 0.01 to 100 wt% of compounds **(A)** and from 0 to 99.99 wt% of compounds **(B).** Preferably the amount of compounds **(A)** expressed this way is from 0.05 to 50 wt% and the amount of compounds **(B)** from 50 to 99.95 wt%. More typically the amount of compounds **(A)** expressed this way is from 0.1 to 30 wt% and the amount of compounds **(B)** from 70 to 99.9 wt%.

Compounds **(B)** typically are (meth)acrylated compounds. (Meth)acrylated compounds **(B)** can be monomers or oligomers, or a mixture of both.

An example of suitable monomers **(B)** are alkyl (meth)acrylates represented by a formula CH2=C(R¹)COOC_{z}H_{2z+1} (wherein R¹ is a hydrogen atom or a methyl group, and z is an integer of from 1 to 13, provided that C_{z}H_{2z+1} may have a straight chain structure or a branched structure), like allyl (meth)acrylate, benzyl (meth)acrylate butoxyethyl (meth)acrylate, butanediol (meth)acrylate, butoxytriethylene glycol mono(meth)acrylate, t-butylaminoethyl (meth)acrylate, 3-chloro-2-hydroxypropyl (meth)acrylate, 2-cyanoethyl (meth)acrylate, cyclohexyl (meth)acrylate, 2,3-dibromopropyl (meth)acrylate, dicyclopentenyl (meth)acrylate, N,N-diethylaminoethyl (meth)acrylate, N,N-dimethylaminoethyl (meth)acrylate, 2-ethoxyethyl (meth)acrylate, 2-(2-ethoxyethoxy)ethyl (meth)acrylate, 2-ethylhexyl (meth)acrylate, glycerol (meth)acrylate, glycidyl (meth)acrylate, heptadecafluorodecyl (meth)acrylate, 2-hydroxyethyl (meth)acrylate, 2-hydroxy-3-(meth)acryloyloxypropyltrimethylammonium chloride, 2-hydroxypropyl (meth)acrylate, [gamma]-(meth)acryloxypropyltrimethoxysilane, 2-methoxyethyl (meth)acrylate, methoxydiethylene glycol (meth)acrylate, methoxytriethylene glycol (meth)acrylate, methoxytetraethylene glycol (meth)acrylate, methoxydipropylene glycol (meth)acrylate, methoxylated cyclodecatriene (meth)acrylate, morpholine (meth)acrylate, nonylphenoxypolyethylene glycol (meth)acrylate, nonylphenoxypolypropylene glycol (meth)acrylate, octafluoropentyl (meth)acrylate, phenoxyhydroxypropyl (meth)acrylate, phenoxyethyl (meth)acrylate, phenoxydiethylene glycol (meth)acrylate, phenoxytetraethylene glycol (meth)acrylate, phenoxyhexaethylene glycol (meth)acrylate, phenoxy (meth)acrylate, polypropylene glycol (meth)acrylate, sodium 2-sulfonate ethoxy (meth)acrylate, tetrafluoropropyl (meth)acrylate, tetrahydrofurfuryl (meth)acrylate, trifluoroethyl (meth)acrylate, hexafluoroisopropyl acrylate, vinyl acetate, N-vinyl caprolactam, N-vinylpyrrolidone, dicyclopentadienyl (meth)acrylate and/or isobornyl acrylate.

Preferred however are monomers with at least 2, more preferably at least 3 polymerizable functional groups such as (meth)acryloyl groups. Examples of poly-unsaturated compounds from this category are trimethylolpropane tri-(meth)acrylate, glycerol tri-(meth)acrylate, pentaerythritol tri,tetra-(meth)acrylate, pentaerythritol tetra-(meth)acrylate, di-trimethylolpropane tetra-(meth)acrylate, di-pentaerythritol hexa-(meth)acrylate and their (poly)ethoxylated and/or (poly)propoxylated equivalents, as well as mixtures thereof. The acrylated forms hereof are preferred. Most preferred are di- and/or tri-acrylates. Examples thereof include but are not limited to: EBECRYL® 145, EBECRYL® 160, trimethylolpropane tri-(meth)acrylate and/or pentaerythritol tri/tetra(meth)acrylate (PETIA) from Cytec.

Preferably, compositions of the invention comprise at least one monomer **(B)** that comprises at least three 3 polymerizable functional groups such as (meth)acryloyl groups.

Preferably compositions of the invention comprise, relative to the total weight of the organic non-volatile content of the composition, at least 10 wt%, more preferably at least 20 wt% and more preferably at least 30 wt% of monomeric compounds **(B),** in casu diluents.

It is also possible to use one or more oligomeric compounds **(B)** selected from: (poly)urethane (meth)acrylates, (poly)ester (meth)acrylates, (poly)ether (meth)acrylates, epoxy (meth)acrylates and/or (meth)acrylic (meth)acrylates. Such compounds are well known in the art.

Preferred are (poly)urethane (meth)acrylates, polyester(meth)acrylates and/or (meth)acrylic (meth)acrylates. Most preferred are (poly)urethane (meth)acrylates and more in particular polyurethane acrylates. By (poly)urethanes is meant to designate urethanes, polyurethanes, and mixtures of both. A polyurethane is meant to designate an organic compound comprising at least 2 carbamate groups. Suitable for use in the present invention are in particular (poly)urethane (meth)acrylates comprising at least 3 (meth)acrylate groups. Preferably the functionality is higher than 3, more preferably higher than 6 or even higher. Suitable for use in the invention are for instance EBECRYL® 1290, EBECRYL® 5129, EBECRYL® 8301, EBECRYL® 8254, EBECRYL® 8405, EBECRYL® 8465 and/or EBECRYL® 8701 from Cytec. Compositions of the invention typically comprise, relative to the total weight of the organic non-volatile content of the composition, from 0 to 90 wt% of such oligomeric compounds **(B).** In general this amount is at least 10 wt% and in general at most 80wt%.

Compositions of the invention may further comprise any known water and/or oil repellency-imparting agent **(C)** which is different from compounds **(A).** Examples include but are not limited to: a fluorine type water and oil repellency-imparting agent using e.g. a fluororesin of e.g. tetrafluoroethylene or vinylidene fluoride, a fluorine compound having a perfluoroalkyl group, a silicone type water and oil repellency-imparting agent using an organopolysiloxane having a siloxane bond in its main chain and having an alkyl group such as a methyl group, an ethyl group or a propyl group; or an organopolysilazane having a silazane bond in its main chain and having an alkyl group such as a methyl group, an ethyl group or a propyl group or a fluoroalkyl group in its side chain, a wax type water and oil repellency-imparting agent using e.g. bees wax or paraffin, or a metal salt type water and oil repellency-imparting agent using a salt of zirconium and a fatty acid, or a salt of aluminum and a fatty acid. Preferred herein are fluorinated compounds and/or silicone compounds **(C).** For instance, compositions of the invention may further comprise one or more silicone compounds and/or fluorinated compounds that are different from compounds **(A).**

When present, compounds **(C)** typically are used in an amount of at most 100 parts by mass per 100 parts by weight of the total weight of compounds **(A).** In general this amount is at most 50, most preferable at most 30 parts by mass of 100 parts by weight of the total weight of compounds **(A).**

Compositions of the invention are preferably curable by ultraviolet irradiation, generally in the presence of photoinitiator **(D).** They can also be cured by electron-beam irradiation, allowing the use of compositions free of photoinitiator. The compositions according to the invention are providing extremely rapid curing.

Photoinitiators **(D)** where present typically are added in an amount of from 0.1 to 10 parts by mass per 100 parts by mass of photopolymerizable compounds. Examples of suitable photoinitiators include but are not limited to an aryl ketone type photoinitiator (such as an acetophenone, a benzophenone, an alkylaminobenzophenone, a benzyl, a benzoin, a benzoin ether, a benzoin dimethyl ketal, a benzoyl benzoate or an [alpha]-acyloxime ester), a sulfur-containing photopolymerization initiator (such as a sulfide or a thioxanthone), an acylphosphine oxide (such as an acyldiarylphosphine oxide) or other photopolymerization initiators. The photopolymerization initiator may be used as a mixture of at least two types thereof in combination. Further, the photopolymerization initiator may be used in combination with a photosensitizer such as an amine.

Compositions of the invention may further comprise, if the case requires, at least one of the following: an ultraviolet absorber, a photostabilizer, an antioxidant, a thermal polymerization inhibitor, a leveling agent, a defoaming agent, a thickener, a sedimentation-preventing agent, a pigment (organic coloring pigment, inorganic pigment), a coloring dye, an infrared absorber, a fluorescent brighter, a dispersant, an antistatic agent, an anti-fogging agent, and/or a coupling agent.

Further, a colloidal silica **(E)** may be added to compositions of the invention to further improve abrasion resistance of the coating film after curing. The average particle size of the colloidal silica **(E)** is not particularly limited, but is preferably from 1 to 1,000 nm, particularly preferably from 1 to 200 nm, especially preferably from 1 to 50 nm, so as to obtain high transparency of the coating film after curing. In order to improve dispersion stability of the colloidal silica (E), the surface of the particles may be modified with a hydrolyzate of a hydrolysable silane compound. Where colloidal silica **(E)** are added, they preferably are added in an (solid content) of at least 0.1 part by mass and at most 500 parts by mass, more preferably at least 1 part by mass and at most 300 parts by mass, particularly preferably at least 10 parts by mass and at most 200 parts by mass, per 100 parts by mass of non-volatile organic content of compound. When the blending amount is within this range, the coating film after curing tends to have sufficient abrasion resistance, haze is less likely to occur on it, and cracks or the like due to an external force are less likely to occur on it.

Organic solvent can be used to reduce viscosity of the coating formulation. The organic solvents typically are removed by heating and drying before cure. In this instance, the heating and drying temperature is preferably, for example, 40° C. or higher to 100° C. or lower. The heating and drying time is, for example, from at least 30 seconds to at most 8 minutes, preferably from at least 1 minute to at most 5 minutes, and more preferably from at least 3 minutes to at most 5 minutes. No particular restriction is given to non-reactive dilution organic solvents that may be used in the invention, including propylene glycol monomethylether acetate, propyleneglycol monomethylether, ethylene glycol monomethylether, butyl acetate, methyl ethyl ketone, methyl isobutyl ketone and isopropyl alcohol - see for instance US 7632874, c14.

If an organic solvent is added at all, then care is taken that it poses no problem in solubility of compounds **(A)** through **(E)** and other additives. It may be any solvent which satisfies the above performances. Further, two or more types of organic solvents may be used in combination. In a case where an organic solvent is added to a composition of the invention, it is preferred to select a proper organic solvent in accordance with the type of the substrate on which a coating film is formed.

Compositions of the invention may be applied in any suitable way known in the art such as spraying, rolling, knife-coating, pouring, brushing, dipping and the like. Also application techniques which will impart micro-nanostructures to the coatings surface are possible like micro- nano gravure, photolithography, UV-nanoimprinting, nano-embossing, micro-replication, electrospinning and the like.

The active energy rays used for curing preferably are ultraviolet rays, electron rays, X-rays, radioactive rays or high frequency waves. Ultraviolet rays having a wavelength of from 180 to 500 nm are particularly preferred from economical viewpoint. Some examples of lamps that may be used include but are not limited to xenon lamps, low-pressure mercury lamps, high-pressure mercury lamps, superhigh-pressure mercury lamps, metal halide lamps, carbon arc lamps or a tungsten lamps, LED lamps, UV-A lamps etc.

Curing time and conditions may vary according to the constituents of the composition, the thickness of the coating film and the active energy ray source used. Usually curing is achieved by irradiation for about 0.1 to about 60 seconds. Further, for the purpose of completing the curing reaction, a heat treatment may be carried out after irradiation with active energy rays. Compositions of the invention typically have a viscosity at 25 degrees Celcius (°C) in the range of from 1 to 50,000 mPa.s. More preferably the viscosity at this temperature is in the range of from 10 to 20,000 mPa.s, most preferably from 50 to 10,000 mPa.s.

Compositions of the inventions advantageously combine an improved compatibility and miscibility with the coating resin matrix and standard additives, with high transparency, fingerprint removability, as well as stain and marker resistance. An advantage of compositions of the invention is that fingerprint removability is long lasting. Not only was it more easy to remove any fingerprints that formed, fingerprints in addition were less likely to attach and caused less damage to the coating surface when wiped with a dry cloth or tissue. Compositions of the invention may further improve anti-corrosion resistance. Non-wettable surfaces may further also impart the ability to prevent frost and/or ice forming or adhering to the surface.

Compositions of the invention further allow to obtain an excellent abrasion resistance.

In contrast to standard coating formulations, coating compositions of the invention showed high transparency, less phase separation and exhibited a good film formation capacity. Compositions of the invention typically have a haze after cure of at most 2 %, more preferably at most 1%, most preferably at most 0.5 %.

Compositions of the invention typically are characterized by a light transmission (T) of at least 80%, more preferably at least 90%, most preferably at least 95%.

Compositions of the invention are highly suited for the making of coating compositions, and more in particular hard coating compositions. The thickness of the hard coat composition typically ranges from 1 to 100 µm. Typically the thickness after curing is at least 1 µm, more typically at least 3 µm, most typically at least 5 µm. Typically the thickness is at most 50 µm, more typically at most 30 µm, most typically at most 25 µm. The coating may be applied in one or more layers depending on the application.

Hard coating compositions according to the invention typically have a pencil hardness after curing as determined by ASTM D3363 of at least H, more in particular at least 2H.

Coating compositions of the invention are further capable of providing a coating film after curing with a haze of at most 2%, more in particular at most 1 %, most in particular at most 0.5%. Coating compositions of the invention are also capable of providing a coating film after curing with a contact angle relative to water on the surface of the coating film of at least 95°, more preferably of at least 100°.

Coating compositions of the invention are further also capable of providing a coating film after curing with a contact angle relative to n-hexadecane on the surface of the coating film of at least 60°, more preferably of at least 62°, most preferably of at least 65°.

Provided in the invention is further a process of coating or treating an article or a substrate, entirely or in part, with a composition of the invention, said process comprising the steps of applying to at least one surface of the article or substrate a composition according to the invention, followed by curing using active energy rays. Typically compositions of the invention are cured using UV irradiation, typically in the presence of a photoinitiator, or using electron beams. Where needed, the curing step may be preceded by a drying step. Where needed, a heat treatment may be carried out after irradiation with the active energy rays.

Typically, any article or substrate coated as such is provided with a coating film that after curing has a contact angle relative to water deposited on the surface of the coating film of at least 95°, more preferably of at least 100°. Typically the contact angle relative to n-hexadecane deposited on the surface of the coating film is at least 60°, more preferably of at least 62°, most preferably more preferably of at least 65°.

Yet another aspect of the invention relates to an article or a substrate coated or treated according to said process.

Coating compositions of the invention may be used in a wide variety of applications and on a wide variety of substrates and articles including molded articles. Coating compositions of the invention are further suited for the making of polymer composite materials or plastic sheets. For instance compositions of the invention may be used on and in satellite dishes, solar energy panels, photovoltaics, exterior architectural glass and green houses, concrete, metal (alloy wheel rims), (automotive) plastic, consumer electronics (mobile phone and PDA displays and showcases; touch screens, large screens and displays (e.g., LCD, CRT, plasma), films (temporary protection self adhesive films for LCDs, solar control windows) and heat transfer surfaces in air conditioning equipment. Other applications include mirror-finished metal plates, glass show windows, showcases, opening materials of automobiles, windshields, mirrors, sunroofs, and headlamps of automobiles and other vehicles, antireflection films, optical filters, optical lenses, displays, projection televisions, plasma displays, EL displays, optical disks, etc. Because of their properties compositions of the invention may further find use in anticorrosive coatings, architectural outdoor applications, as well as indoor and outdoor automotive applications. Since a coating of the invention also provides a low coefficient of friction, it may also be applied on pipes and tubes being used for liquid or gas transport.

Substrates that may be treated or coated with compositions of the invention include metal, wood, paper, concrete, plastics (porous and non-porous), glass, as well as coating surfaces. Articles or materials to which the coating composition is applied may for instance already contain one or more coating layers (e.g. articles or material may already contain a primer or a base coat).

Besides a use as coating compositions, the compositions of the invention may further be used for the making of inks, varnishes and adhesives.

Provided are also coatings compositions, inks, varnishes and adhesives that are prepared from, or that comprise, a composition of the invention.

Another aspect of the invention relates to the use of a composition of the invention for the making of a coating film that after curing has a contact angle relative to water on the surface of the coating film of at least 95°, more preferably of at least 100°.

Yet another aspect of the invention relates to the use of a composition of the invention for the making of a coating film that after curing has a contact angle relative to n-hexadecane on the surface of the coating film of at least 60°, more preferably of at least 62°, most preferably of at least 65°.

Still a further aspect of the invention relates to the use of a composition of the invention for the making of a transparent coating that after cure has a light transmission (T) of >90%.

Throughout the Invention and in the Examples Section the following measuring methods have been used to characterize the compounds and compositions of the invention as well as coatings obtained herewith:
Molecular weight determination via GPC: A small portion of sample is dissolved in tetrahydrofuran (THF) and injected into a liquid chromatograph (Merck-Hitachi L7100) equipped with 4 PLGeI Mixed-A polystyrene divinylbenzene GPC columns (300mm X 7.5mm X 20µm). Typically polystyrene standards (typically in the Molecular Weight range: 200 - 7.500.000 Daltons) are added as internal standards. The components of the sample are separated by the GPC columns based on their molecular size in solution and detected by a Refractive Index detector. Data typically are gathered and processed by Polymer Laboratories Cirrus GPC software.
Viscosity (cone plate): viscosity was measured with a rotational viscometer at 25°C with defined shear rate of 20 s-1, according to DIN EN ISO 3219. The viscosity value is expressed in mPa.s
Contact Angle: The contact angle of water or n-hexadecane droplets was determined using a OCA20 station of DataPhysics equipped with a CCD videocamera. For this purpose, sessile drops were formed on the substrate using an automated syringe fitted with a teflonated circular steel needle (Hamilton) inserted in the drop. The drop volume was increased to 10 and 15 µL at a steady rate of 1 µL s-1. Next the needle was slowly removed from the drop while recording the image sequence using a digital video system ("frame grabber"). The digital drop images were subsequently analysed and the shape was fitted to the Laplace-Young equation in order to determine the contact angle. Average CA values of two drops (10 and 15 µL) are reported.
Fingerprint Removability: The fingerprints attached to the surface of a sample coating film were wiped off by a dry cloth, and the removability was visually judged.

The evaluation standards were as follows:
○: Fingerprint completely removable.
X : Fingerprint not removable.

Transparency of coated films: The haze (%) is measured on four points of a 200 µm polycarbonate film coated with a 10 µm thick coating layer with a haze meter (XL-211 Hazegard Hazemeter from BYK Gardner). Average haze values measured on 4 different points on the coated substrate are calculated.

Residual lactide content: The residual lactide content was measured via ¹H-NMR (Spectrometer: Bruker Avance 300) using CDCl₃ as solvent. The mole % free lactide vs polymerized lactide is determined by integration of the ring lactide methyl protons (doublet with chemical shift at δ 1.65) and the methyl-groups the ringopened polymerized lactide (broadened doublets from δ 1.49 to 1.60).

The invention is now further described in more details in the following Examples, which in no way intend to limit the invention or its applications.

### Preparative Example 1 P: Adduct lactide (2)/2-hydroxvethyl acrylate

500 g of the lactide (FUTERRO® Lactide LF from Futerro), 207 g 2-hydroxyethylacrylate, 0.35 g of hydroxyquinone monomethyl ether (MeHQ) and 0.71 g of Sn-T9 stannous octoate (T9) were placed into a reaction flask equipped with an agitator, liquid addition funnel and thermometer. The reaction mixture was heated to 130°C and stirred until the residual lactide was lower than 5 mole %. A product with a viscosity (Höppler, ISO 12058 at 25°C) of 1376 mPa.s was obtained.

### Preparative Example 2P: Adduct lactide (2,5)/2-hydroxyethyl acrylate

600 g of the lactide (FUTERRO® Lactide LF from Futerro), 185,6 g 2-hydroxyethylacrylate, 0.38 g of hydroxyquinone monomethyl ether (MeHQ) and 0.76 g of Sn-T9 stannous octoate (T9) were placed into a reaction flask equipped with an agitator, liquid addition funnel and thermometer. The reaction mixture was heated to 130°C and stirred until the residual lactide was lower than 5 mole %. A product with a viscosity (Höppler, ISO 12058 at 25°C) of 13108 mPa.s was obtained.

### Preparative Example 3P: Adduct lactide (3)/2-hydroxyethyl acrylate

1000 g of the lactide (FUTERRO® Lactide LF from Futerro), 286.6 g 2-hydroxyethylacrylate, 0.6 g of hydroxyquinone monomethyl ether (MeHQ) and 1.3 g of Sn-T9 stannous octoate (T9) were placed into a reaction flask equipped with an agitator, liquid addition funnel and thermometer. The reaction mixture was heated to 130°C and stirred until the residual lactide was lower than 5 mole %. A product with a viscosity (Höppler, ISO 12058 at 25°C) of 36590 mPa.s was obtained.

### Preparative Example 4P: adduct lactide(4)/2-hydroxyethyl acrylate

800 g of the lactide, 161.1 g 2-hydroxyethylacrylate, 0.5 g of hydroxyquinone monomethyl ether (MeHQ) and 1.92 g of dibutyltindilaurate (DBTL) were placed into a reaction flask equipped with an agitator, liquid addition funnel and thermometer. The reaction mixture was heated to 130°C and stirred until the residual lactide was lower than 5 %. A product with a viscosity (Höppler, ISO 12058 at 60°C) of 1588 mPa.s was obtained.

### Synthetic Example SE-3

700 g of the Fluorolink E10H, 0.45 g of trisphenylphosphite (TPP), 0.13 g of butylated hydroxytoluene (BHT), 206.5 g of isophorone diisocyanate and 0.39 g of dibutyltindilaurate (DBTL) were placed into a reaction flask equipped with an agitator, liquid addition funnel and thermometer. The reaction mixture was heated to 90°C and stirred until the NCO content was about 4.52 %. The addition funnel was fed with a mixture of 388.7 g of adduct from lactide with 2-hydroxylethyl acrylate (Example 1 P) and 0.4 g of DBTL which was added in 2 hours. The reaction mixture was maintained at 80°C until the residual NCO content was lower than 0.2 %. The product was finally diluted with 3855 g PETIA. A clear product with a viscosity (Höppler, ISO 12058 at 25°C) of 7580 mPas was obtained.

### Synthetic Example SE-4

550 g of the Fluorolink D10H, 0.31 g of butylated hydroxytoluene (BHT), 173.9 g of isophorone diisocyanate and 0.31 g of dibutyltindilaurate (DBTL) were placed into a reaction flask equipped with an agitator, liquid addition funnel and thermometer. The reaction mixture was heated to 90°C and stirred until the NCO content was about 4.43 %. The addition funnel was fed with a mixture of 323.9 g of adduct from lactide with 2-hydroxylethyl acrylate (Example 1P). The reaction mixture was maintained at 80°C until the residual NCO content was lower than 0.2 %. The product was finally diluted with 3143 g PETIA. An clear product with a viscosity (Höppler, ISO 12058 at 25°C) of 6840 mPa.s was obtained.

### Synthetic Example SE-5

500 g of the Fluorolink D10H, 0.28 g of butylated hydroxytoluene (BHT), 132.2 g of isophorone diisocyanate and 0.28 g of dibutyltindilaurate (DBTL) were placed into a reaction flask equipped with an agitator, liquid addition funnel and thermometer. The reaction mixture was heated to 90°C and stirred until the NCO content was about 3.97 %. The addition funnel was fed with a mixture of 292.7 g of adduct from lactide with 2-hydroxylethyl acrylate (Example 2P). The reaction mixture was maintained at 80°C until the residual NCO content was lower than 0.2 %. The product was finally diluted with 2774 g PETIA. An clear product with a viscosity (Höppler, ISO 12058 at 25°C) of 7154 mPa.s was obtained.

### Synthetic Example SE-6

500 g of the Fluorolink D10H, 0.29 g of butylated hydroxytoluene (BHT), 132.2 g of isophorone diisocyanate and 0.29 g of dibutyltindilaurate (DBTL) were placed into a reaction flask equipped with an agitator, liquid addition funnel and thermometer. The reaction mixture was heated to 90°C and stirred until the NCO content was about 3.97 %. The addition funnel was fed with a mixture of 327.4 g of adduct from lactide with 2-hydroxylethyl acrylate (Example 3P). The reaction mixture was maintained at 80°C until the residual NCO content was lower than 0.2 %. The product was finally diluted with 2897 g PETIA. An clear product with a viscosity (Höppler, ISO 12058 at 25°C) of 7328 mPa.s was obtained.

### Synthetic Example SE-7

550 g of the Fluorolink D10H, 0.39 g of butylated hydroxytoluene (BHT), 173.9 g of isophorone diisocyanate and 0.39 g of dibutyltindilaurate (DBTL) were placed into a reaction flask equipped with an agitator, liquid addition funnel and thermometer. The reaction mixture was heated to 90°C and stirred until the NCO content was about 4.67 %. The addition funnel was fed with a mixture of 568.4 g of adduct from lactide with 2-hydroxylethyl acrylate (Example 4P). The reaction mixture was maintained at 80°C until the residual NCO content was lower than 0.2 %. The product was finally diluted with 3877 g PETIA. A clear product with a viscosity (Höppler, ISO 12058 at 25°C) of 7517 mPa.s was obtained.

### Synthetic Example SE-8

500 g of the Fluorolink D10H, 152 g lactide, 0.98 g of dibutyltindilaurate (DBTL) were placed into a reaction flask equipped with an agitator, liquid addition funnel and thermometer. The reaction mixture was heated to 120°C and stirred until the lactide-value was lower than 0.5 %. The reaction product was cooled down to 30°C and 115.6 g of isophorone diisocyanate together with 0.61 g MeHQ was added. The reaction mixture was heated to 90°C and stirred until the NCO content was about 2.78 %. The addition funnel was fed with a mixture of 2955 g of PETIA and 0.38 g of DBTL which was added in 2 hours. The reaction mixture was maintained at 80°C until the residual NCO content was lower than 0.2 %. A product composition with a viscosity (Höppler, ISO 12058 at 25°C) of 9533 mPa.s was obtained.

### Synthetic Example SE-9

500 g of the Fluorolink E10H, 100.89 g of lactide and 0.6 g of dibutyltindilaurate (DBTL) were placed into a reaction flask equipped with an agitator, liquid addition funnel and thermometer. The reaction mixture was heated to 130°C and stirred until the free lactide content was below 5 %. The reaction mixture was cooled down to 50°C and 66.7 g of succinic anhydride, 0.53 g of hydroquinone (HQ) and 1.14 g of Hycat OA were added. The reaction mixture was heated to 110°C until [lac - lac(total)] was below 11 mg KOH/g. The addition funnel was fed with 94.7 g glycidylmethacrylate (GMA) acrylate which was added in 2 hours. The reaction mixture was maintained at 110°C until the acid value was below 5 mg KOH/g and epoxy value below 0.2 %. The product was finally diluted with 2287 g PETIA. A slightly hazy product with a viscosity (Höppler, ISO 12058 at 25°C) of 3386 mPa.s was obtained.

### Comparative Example 1-RC

606.3 g of the Fluorolink E10H, 0.38 g of trisphenylphosphite (TPP), 0.11 g of butylated hydroxytoluene (BHT), 179.3 g of isophorone diisocyanate and 0.3 g of dibutyltindilaurate (DBTL) were placed into a reaction flask equipped with an agitator, liquid addition funnel and thermometer. The reaction mixture was heated to 90°C and stirred until the NCO content was about 4.81 %. The addition funnel was fed with a mixture of 94 g of 2-hydroxylethyl acrylate and 0.3 g of DBTL which was added in 2 hours. The reaction mixture was maintained at 75°C until the residual NCO content was lower than 0.1 %. After addition of 2639 g PETIA a product composition with a viscosity (Höppler, ISO 12058 at 25°C) a whitely cloudy product with a viscosity (Höppler, ISO 12058 at 25°C) of 2465 mPa.s was obtained.

### Examples 1 to 14 and Comparative Examples 1-R to 3-R

Oligomers as prepared according to Synthetic Examples SE-3 to SE-9 and the Comparative Example 1-RC were tested in two different concentrations in a base coating formulation. The formulations were made by mixing the product examples with a urethane acrylate : EBECRYL® 8301 (available from Cytec), acrylated monomers : EBECRYL® 145 (available from Cytec) and pentaerythritol tri/tetra acrylate PETIA (available from Cytec); and initiator Additol CPK (available from Cytec). Information on the compositions of the base formulation tested and characteristics thereof are given in Table 1 below. A comparison was also made with a standard hard coat formulation - Comparative Example 1-R.

**Table 1: Composition of the Base Formulations (in grams)**

| | Formulation 1 | Formulation 2 |
|---|---|---|
| Ebecryl 8301 | 30 | 27 |
| PETIA | 33 | 30 |
| Ebecryl145 | 21 | 19 |
| Additol BCPK | 4 | 4 |
| Oligomers tested* | 12 | 20 |

| | | |
|---|---|---|
| * Oligomers are diluted in 75 % PETIA. | | |

The results show an improved compatibility when using compounds (A) of the invention compared to compounds that lack a lactide modification (Comparative Examples 2-R and 3-R). This follows clearly from Table 2 below.

### Applications and testing:

UV formulations were applied by means of a 10 µm bar coater on a transparent sheet made of polycarbonate resin with a thickness of 0.2 mm and exposed to UV radiations from a 120 W/cm non focalized medium pressure mercury vapor lamp at 2 x 10 m/min.

The samples were tested for coating aspect, transparency (haze), water contact angle (before and after 200 ADR - acetone double rubs), marker resistance and fingerprint removability. The results obtained are presented in Table 3 below.

**Table 2 (in grams):**

| | **Ex1-R** | **Ex 2-R** | **Ex 3-R** | **Ex1** | **Ex2** | **Ex3** | **Ex4** | **Ex5** | **Ex6** | **Ex7** | **Ex8** | **Ex9** | **Ex10** | **Ex11** | **Ex12** | **Ex13** | **Ex14** |
|---|---|---|---|---|---|---|---|---|---|---|---|---|---|---|---|---|---|
| EEBECRYL ®8301 | 34 | 30 | 27 | 30 | 30 | 30 | 30 | 30 | 30 | 30 | 27 | 27 | 27 | 27 | 27 | 27 | 27 |
| PETIA | 38 | 33 | 30 | 33 | 33 | 33 | 33 | 33 | 33 | 33 | 30 | 30 | 30 | 30 | 30 | 30 | 30 |
| EBECRYL® 145 | 24 | 21 | 19 | 21 | 21 | 21 | 21 | 21 | 21 | 19 | 19 | 19 | 19 | 19 | 19 | 19 | 19 |
| ADDITOL® BPCK | 4 | 4 | 4 | 4 | 4 | 4 | 4 | 4 | 4 | 4 | 4 | 4 | 4 | 4 | 4 | 4 | 4 |
| 1RC | | 12 | 20 | | | | | | | | | | | | | | |
| SE-3 | | | | 12 | | | | | | | 20 | | | | | | |
| SE-4 | | | | | 12 | | | | | | | 20 | | | | | |
| SE-5 | | | | | | 12 | | | | | | | 20 | | | | |
| SE-6 | | | | | | | 12 | | | | | | | 20 | | | |
| SE-7 | | | | | | | | 12 | | | | | | | 20 | | |
| SE-8 | | | | | | | | | 12 | | | | | | | 20 | |
| SE-9 | | | | | | | | | | 12 | | | | | | | 20 |
| Fluorinated oligomer content (wt%) | 0 | 3 | 5 | 3 | 3 | 3 | 3 | 3 | 3 | 3 | 5 | 5 | 5 | 5 | 5 | 5 | 5 |
| Viscosity (25°C, mPa.s) | 830 | 890 | 1095 | 1125 | 1055 | 1075 | 1083 | 1090 | 1050 | 1035 | 1400 | 1310 | 1344 | 1365 | 1380 | 1305 | 1190 |
| Formulation aspect | clear | whitely cloudy/ opaque | whitely cloudy/ opaque | clear | clear | clear | clear | clear | opaq. | clear | clear | clear | clear | clear | clear | clear | clear |

**Table 3:**

| | **Ex1-R** | **Ex 2-R** | **Ex 3-R** | **Ex1** | **Ex2** | **Ex3** | **Ex4** | **Ex5** | **Ex6** | **Ex7** | **Ex8** | **Ex9** | **Ex10** | **Ex11** | **Ex12** | **Ex13** | **Ex14** |
|---|---|---|---|---|---|---|---|---|---|---|---|---|---|---|---|---|---|
| Coating aspect | OK | Orange Peel & Pinholes | Orange Peel & Pinholes | OK | OK | OK | OK | OK | OK | OK | OK | OK | OK | OK | OK | OK | OK |
| Coating haze (%) | 0,2 | 10,4 | 15,6 | 0,3 | 0,2 | 0.2 | 0.3 | 0,2 | 2.4 | 0,2 | 0,1 | 0,1 | 0.2 | 0.2 | 0,2 | 0,2 | 0,2 |
| Water contact angle (°) | 71.1 | - | - | 98.7 | 110.8 | 109.0 | 105.3 | 110.8 | 109.5 | 103.9 | 108.6 | 110.4 | 107.7 | 108.5 | 111.2 | 109.2 | 105.6 |
| Hexadecane contact angle (°) | <10 | - | - | 60,5 | 69,1 | 67.7 | 68.8 | 70,6 | 68,5 | 64,2 | 67,4 | 67,9 | 67.0 | 67.9 | 68,4 | 66,9 | 66,1 |
| Marker resistance | 5 | - | - | 1 | 1 | 1 | 1 | 1 | 1 | 1 | 1 | 1 | 1 | 1 | 1 | 1 | 1 |
| Fingerprint removability | X | - | - | O | O | O | O | O | O | O | O | O | O | O | O | O | O |

| | | | | | | | | | | | | | | | | | |
|---|---|---|---|---|---|---|---|---|---|---|---|---|---|---|---|---|---|
| - not determined | | | | | | | | | | | | | | | | | |

## Claims

1. An active energy ray curable compound **(A)** comprising: at least one active energy ray curable group, at least one fluorine-containing moiety **(a1)** and at least one moiety **(a2)** comprising at least one portion represented by Formula (1):
-[C(=O)-CH(R)-O-C(=O)-CH(R)-O]n-
wherein n is an integer from 1 to 10, and wherein R is selected from -H or -CH₃.

2. The compound of claim 1, wherein moiety **(a1)** is a fluorinated alcohol-derived moiety, wherein the fluorinated alcohol preferably is selected from fluorinated oxetane alcohols, hydroxyl functional fluoroethylene-alkyl vinyl ethers, fluoroalkyl alcohols and/or fluorinated (poly)ether alcohols.

3. The compound of any of the preceding claims, wherein moiety **(a1)** comprises at least one portion represented by one or more of the Formulae (2) to (6):
-(CF₂CF₂O)p- Formula (2)
-(CF₂CF(CF₃)0)q-; Formula (3)
-(CF₂CF₂CF₂0)r- Formula (4)
-(CF₂O)s- Formula (5);
-(CkF2k+1)- Formula (6);
wherein k is an integer from 1 to 16, and each of p, q, r and s is an integer of from 1 to 100.

4. The compound of any of the preceding claims, wherein the active energy ray curable functional group is selected from: (meth)acryloyl groups, vinyl groups, allyl groups, thiol groups and/or epoxy groups.

5. The compound of any of claims 1 to 4, obtained from the reaction of:
- at least one fluorinated alcohol **(i),**
- at least one polyisocyanate **(ii),** and
- at least one (poly)lactide- and/or (poly)glycolide-modified compound **(iii)** containing at least one active energy ray curable group and essentially one reactive group capable to react with isocyanate groups.

6. The compound of claim 5, wherein compounds **(iii)** are represented by Formula (9):
CH2=C(R')C(=O)O(CrH2rO)t-[C(=O)-CH(R)-O-C(=O)-CH(R)-O]ₙ-H
wherein r is an integer from 1 to 4, t is an integer from 1 to 4, n is an integer from 1 to 10, and wherein each of R and R', independently, is selected from -H or -CH₃.

7. The compound of any of claims 1 to 4, obtained from the reaction of:
- at least one fluorinated alcohol **(i'),**
- at least one polyisocyanate **(ii'),**
- at least one lactide and/or glycolide **(iii'),** and
- at least one compound **(vi')** containing at least one active energy ray curable group and essentially one reactive group capable to react with isocyanate groups.

8. The compound of any of claims 1 to 4, obtained from the reaction of:
- at least one fluorinated alcohol **(i'),**
- at least one lactide and/or glycolide **(iii'),**
- at least one anhydride **(vii'),** and at least one compound **(viii')** containing at least one active energy ray curable group and essentially one reactive group capable to react with carboxylic acid groups.

9. The compound of claim 8, wherein compound **(viii')** is a (meth)acrylate containing essentially one group selected from hydroxyl groups, epoxy groups and/or amino groups.

10. The compound of any of claims 5 to 9, wherein the fluorinated alcohol is a polyfluoro (poly)ether alcohol, more in particular a perfluoro (poly)ether diol.

11. An active energy ray curable composition comprising, relative to the total weight of non-volatile parts of the composition, from 0,5 to 99.5% by weight of at least one compound **(A)** of any of claims 1 to 10, and from 0 to 99,5% by weight of at least one active energy ray curable compound **(B).**

12. The composition of claim 11, wherein the composition comprises from 3 to 90% by weight of compounds **(A).**

13. The composition of any of claims 11 to 12, wherein compounds **(B)** are selected from: (poly)urethane (meth)acrylates, (poly)ester (meth)acrylates, (poly)ether (meth)acrylates, epoxy (meth)acrylates and/or (meth)acrylated (meth)acrylics.

14. The composition of any of claims 11 to 13, further comprising at least one compound **(C)** selected from silicone compounds and/or from fluorinated compounds different from **(A).**

15. A process of coating an article or a substrate, comprising the steps of applying to at least one surface of an article or a substrate a composition of any of claims 11 to 14, followed by curing.

16. The process of claim 15, wherein the haze of said coating after cure is at most 1.0 % and the thickness of said coating after cure is in the range of from 5 to 25 µm.

17. An article coated according to the process of any of claims 15 or 16, wherein the coated article is **characterized by** one or more of the following:
- a contact angle relative to water deposited on the coated surface of at least 95°, preferably at least 100°;
- a contact angle relative to n-hexadecane deposited on the coated surface of at least 60°, more preferably at least 62°, most preferably at least 65°.
